(19) Europäisches Patentamt
European Patent Office
Office européen des brevets

(11) Veröffentlichungsnummer: **0 094 605**
**B1**

# (12) EUROPÄISCHE PATENTSCHRIFT

(45) Veröffentlichungstag der Patentschrift: **10.12.86**

(21) Anmeldenummer: **83104618.0**

(22) Anmeldetag: **11.05.83**

(51) Int. Cl.⁴: **C 07 D 519/00,** C 07 D 498/10, C 08 K 5/34 // (C07D498/10, 263:00, 221:00), (C07D519/00, 498:00, 498:00)

## (54) Polyalkyldiazaspirodecanderivate, Verfahren zu ihrer Herstellung und ihre Verwendung als Stabilisatoren.

(30) Priorität: **14.05.82 DE 3218172**

(43) Veröffentlichungstag der Anmeldung: **23.11.83 Patentblatt 83/47**

(45) Bekanntmachung des Hinweises auf die Patenterteilung: **10.12.86 Patentblatt 86/50**

(84) Benannte Vertragsstaaten: **AT BE CH DE FR GB IT LI NL SE**

(56) Entgegenhaltungen:
**EP - A - 0 017 617**
**EP - A - 0 022 997**
**DE - A - 2 933 732**

(73) Patentinhaber: **HOECHST AKTIENGESELLSCHAFT,** ***Postfach 80 03 20, D-6230 Frankfurt am Main 80 (DE)***

(72) Erfinder: **Wiezer, Hartmut, Dr., Am Stocket 18, D-8901 Lützelburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 094 605 B1

ACTORUM AG

**Beschreibung**

In der Literatur sind zahlreiche, auf die unterschiedlichste Weise modifizierte oder substituierte Polyalkyldiazaspirodecane beschrieben (vgl. z.B. DE-A 2 606 026, 2 634 957 und 2 834 962, DE-A 2 933 732 und EP-A 17 617). Den Verbindungen ist gemeinsam, dass die Wasserstoffatome der die 2-Position des Oxazolidinonringes einnehmenden $CH_2$-Gruppe zwar gegebenenfalls substituiert sein können, jedoch nicht durch funktionelle Gruppen tragen Alkylreste.

Die Erfindung betrifft daher neue, in 2-Position des Ringsystems durch einen funktionelle Gruppen tragenden Alkylrest substituierte Polyalkyldiazaspirodecanderivate, ein Verfahren zu ihrer Herstellung und ihre Verwendung als Stabilisatoren für Kunststoffe.

Die erfindungsgemässen Verbindungen entsprechend der Formel (I),

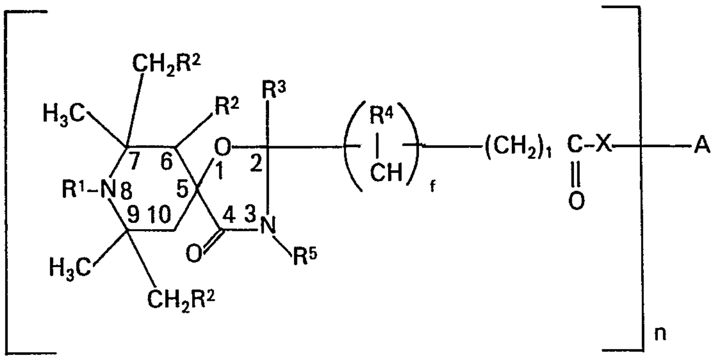

(I),

in welcher

$R^1$ Wasserstoff, Sauerstoff, $C_1$- bis $C_{12}$-, vorzugsweise $C_1$- bis $C_4$-Alkyl, das durch 1 OH substituiert sein kann, oder 2,3-Dihydroxipropyl, insbesondere jedoch Wasserstoff ist,

$R^2$ Wasserstoff oder $C_1$- bis $C_5$-Alkyl, vorzugsweise Wasserstoff oder Methyl und insbesondere Wasserstoff darstellt,

$R^3$ eine $C_1$- bis $C_5$-Alkylgruppe, die durch $-COOH$, $-COOCH_3$ oder $-COOC_2H_5$ substituiert sein kann, vorzugsweise eine $C_1$- oder $C_2$-Alkylgruppe, oder eine Phenylgruppe bedeutet, und

$R^4$ dann Wasserstoff ist oder

$R^3$ und $R^4$ zusammen mit den sie bindenden Kohlenstoffatomen einen $C_5$- bis $C_{12}$-, vorzugsweise $C_6$-Cycloalkanring bilden.

f ist null oder 1, vorzugsweise 1,

1 hat die Bedeutung einer ganzen Zahl von 0 bis 10, vorzugsweise von 0 bis 7,

n bedeutet eine ganze Zahl von 1 bis 10, vorzugsweise 1 bis 6 und insbesondere 1 bis 4,

$R^5$ ist Wasserstoff, eine $C_1$- bis $C_{12}$-Alkylgruppe, die in 2-Stellung durch OH substituiert sein kann, oder eine Gruppe der Formel $-(CH_2)_kCOOR^6$, worin k = 1 oder 2 ist und $R^6$ für eine $C_1$- bis $C_{18}$-, vorzugsweise $C_1$- oder $C_2$-Alkylgruppe steht.

X die Bedeutung von -O- oder

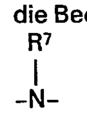

hat, wobei $R^7$ die unten für A bei n = 1 angegebenen Bedeutungen hat.

A steht

bei n = 1 für Wasserstoff, $C_1$- bis $C_{18}$-, vorzugsweise $C_1$- bis $C_6$-Alkyl, für $C_3$- bis $C_7$-Alkenyl, für $C_7$- bis $C_9$-Phenylalkyl, für $C_5$- bis $C_{12}$-Cycloalkyl oder für 2,2,6,6-Tetramethyl-4-piperidinyl,

bei n = 2 für einen $C_2$- bis $C_{30}$-, vorzugsweise $C_2$- bis $C_{18}$- und insbesondere $C_2$- bis $C_6$-Alkylenrest, für einen $C_4$- bis $C_8$-Alkenylenrest oder für einen Mono-, Di- oder Tricycloalkylenrest mit 6 bis 18, vorzugsweise 6 bis 12 C-Atomen,

bei n = 3 für einen $C_3$- bis $C_8$-, vorzugsweise $C_3$- bis $C_5$-Alkantriylrest, für einen Rest der Formel

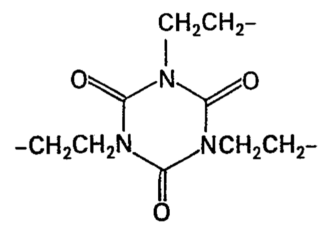

oder für eine Gruppe, die zusammen mit X eine Gruppe der Formel

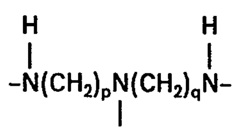

worin p und q gleich oder verschieden und 2 oder 3 darstellen, bedeutet,

bei n = 4 für eine $C_4$- bis $C_8$-, vorzugsweise $C_5$-Alkantetraylgruppe oder mit X zusammen für eine Gruppe der Formel

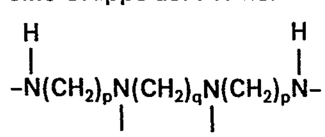

in der p = 2 oder 3 und q = 2 ist, und

bei n >4 für einen $C_5$- bis $C_{10}$-Alkanrest, der eine Ethergruppe enthalten kann und sich von einem, nicht zwei OH-Gruppen pro C-Atom tragenden Polyalkohol ableitet, oder mit X zusammen für einen Polyethylenpolyamin-Rest.

Beispiele für unter die Formel (I) fallende Verbindungen sind:

1. 2,7,7,9,9-Pentamethyl-1-oxa-3,8-diaza-4-oxo-spiro-[4,5]-decan-2-yl-essigsäureethylester
2. 2,7,7,9,9-Pentamethyl-1-oxa-3,8-diaza-4-oxo-spiro-[4,5]-decan-2-yl-propionsäureethylester
3. 2,7,7,9,9-Pentamethyl-1-oxa-3,8-diaza-4-oxo-spiro-[4,5]-decan-2-yl-butancarbonsäuremethylester
4. 2,2,4,4-Tetramethyl-7-oxa-3,14-diaza-15-oxo-dispiro-[5.1.5.2]-pentadecan-9-yl-propionsäurepropylester
5. 1,6-Hexan-bis-[(2,7,7,9,9-Pentamethyl-1-oxa-3,8-diaza-4-oxo-spiro-[4,5]-decan-2-yl)-propionsäureamid]
6. 1,6-Hexamethylendi-[(2,7,7,9,9-pentamethyl-1-oxa-3,8-diaza-4-oxo-spiro-[4,5]-decan-2-yl)-ethylencarboxylat]
7. 1,3,5-Ethylen-2,4,6-trioxo-1,3,5-triazin-tris-[(2,7,7,9,9-pentamethyl-1-oxa-3,8-diaza-4-oxo-spiro-[4,5]-decan-2-yl)-ethylenylcarboxylat]
8. 1,2-Ethylendioxi-bis-(2,7,7,9,9-pentamethyl-1-oxa-3,8-diaza-4-oxo-spiro-[4,5]-decan-2-yl)-ethylcarboxylat
9. 2,2,4,4-Tetramethyl-7-oxa-3,14-diaza-15-oxo-dispiro-[5.1.5.2]-pentadecan-9-yl-propionsäureamid

Die neuen Verbindungen werden entsprechend dem nachstehenden Reaktionsschema durch Umsetzen von Polyalkylpiperidincyanhydrinen (II) mit der 1- bis 2fach, vorzugsweise der im Hinblick auf Ketogruppen, äquivalenten Menge einer Ketonverbindung der Formel (III) bei 40 bis 120, vorzugsweise 60 bis 100 und insbesondere 70 bis 100°C in einer $C_1$- bis $C_5$-Mono-Carbonsäure, vorzugsweise Essigsäure als Lösungsmittel in Gegenwart der, bezogen auf Cyanhydrin, 1,5- bis 5fach-, vorzugsweise 2,0- bis 3fach- und insbesondere 2,0- bis 3,5fach molaren Menge einer nicht oxidierenden anorganischen Säure, vorzugsweise HCl oder HBr und insbesondere HCl in Form eines Mineralsäuresalzes erhalten, welches man anschliessend in die freien Basen umwandelt (Weg A).

Will man die Verbindungen mit n >1 herstellen, so kann man vorzugsweise derart vorgehen, dass man zunächst wie vorstehend beschrieben, nach Weg A aus (II) und (IIIa) Verbindungen mit X = –O– und A = $-CH_3$ oder $C_2H_5$ synthetisiert (I a) und diese sodann mit der äquivalenten Menge eines Amins oder Alkohols der Formel (IV) in inerten organischen Lösungsmitteln in Gegenwart von 0,1 bis 1%, bezogen auf (I a), eines Katalysators wie z.B. $LiNH_2$, NaH, $NaOCH_3$ oder auch $Ti(OC_3H_7)_4$ unter Methanol- oder Ethanolabspaltung umsetzt (Weg B).

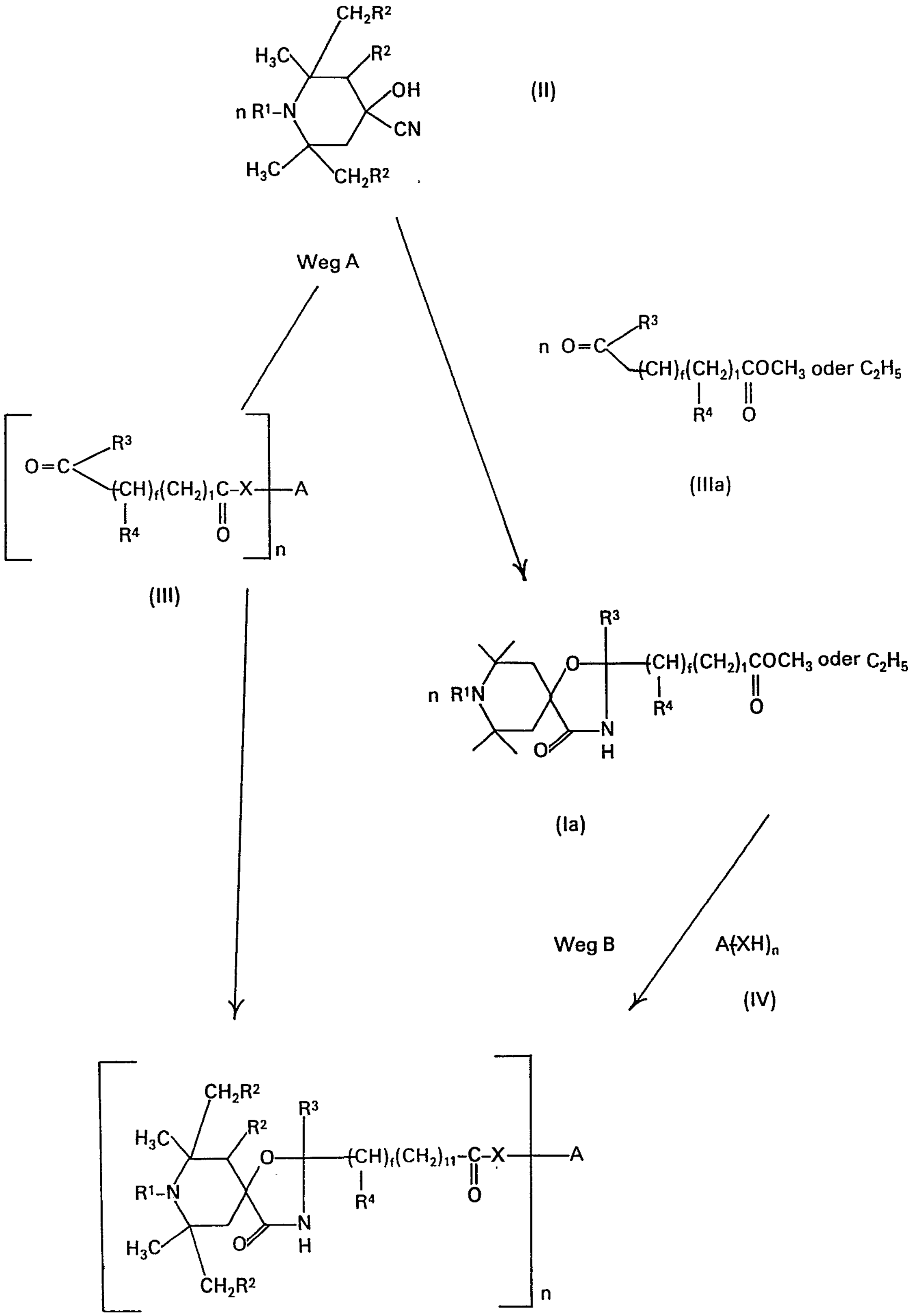


(II)

(III)

(IIIa)

(Ia)

(IV)

(I)

In den Formeln (II), (III), (IIIa), (IV), (I) und (Ia) haben $R^1$, $R^2$, $R^3$, $R^4$, f, I, X, n und A die oben angegebenen Bedeutungen und steht n in Formel (III) vorzugsweise für 1 oder 2.

Falls gewünscht, können die Verbindungen (I) oder (Ia) durch Umsetzen mit der, bezogen auf Lactam-NH-äquivalenten Menge einer Halogenverbindung oder eines Epoxids dann noch zu Produkten (I) mit $R^5 \neq H$ derivatisiert werden.

Die für die Umsetzung benötigten Polyalkylpiperidylcyanhydrine sind nach den Angaben der DE-OS 2 830 719 herstellbar in Anlehnung an US-PS 2 259 167.

Als Ketonverbindungen seien beispielsweise genannt:
Lävulinsäureethylester, Brenztraubensäureethylester, Acetessigsäureethylester, 5-Oxohexansäureethylester, Cyclohexanon-2-propionsäureethylester und Acetessigsäureglykolester.

Dass sich die neuen Verbindungen in der beschriebenen Weise herstellen lassen würden, war nicht vorhersehbar, weil damit gerechnet werden musste, dass die als Reaktionskomponente dienenden substituierten Ketona, insbesondere wenn es sich um Estergruppen enthaltende handelt, in oder mit dem sauren Reaktionsmedium zu Nebenreaktionen neigen würden.

Die neuen Verbindungen zeichnen sich gegenüber denen des Standes der Technik durch eine sehr gute Wirksamkeit, verbunden mit einem besonders günstigen anwendungstechnischen Gesamtbild, aus. Ein weiterer Vorteil besteht darin, dass höhere Molgewichte sehr leicht durch Modifizierung des funktionellen Restes erhalten werden können, was bei den Verbindungen des Standes der Technik nicht ohne weiteres möglich ist. Derartige Vorteile durch Einführung eines funktionellen Restes erzielen zu können, liess sich nicht vorhersehen und muss als überraschend bezeichnet werden.

Die neuen, durch funktionelle Gruppen substituierten Polyalkyldiazaspirodecane sind hervorragend zum Stabilisieren von organischen Polymeren geeignet, wie sie beispielsweise in der DE-OS 3 045 839 auf den Seiten 14 bis 19 aufgeführt sind, wobei auch die dort zitierten weiteren Additive zugesetzt werden können. Ihre Einsatzmenge beträgt im allgemeinen 0,01 bis 5, vorzugsweise 0,05 bis 2,5 und insbesondere 0,1 bis 1 Gewichtsteile, bezogen auf das Polymere.

Besonders brauchbar sind die neuen Verbindungen zum Stabilisieren von einem Polyolefin, z.B. Polypropylen, Polyethylen sowie Polystyrol, ABS-Harzen, Poly(meth)acrylaten und Copolymeren der entsprechenden Monomeren geeignet; ein weiteres bevorzugtes Einsatzgebiet ist für Produkte der Formel (I), in denen $R^5$ nicht H ist, der Lacksektor.

Die nachstehenden Beispiele dienen der weiteren Erläuterung der Erfindung.

In den folgenden Herstellungsbeispielen sind die Verfahrensprodukte durch Ziffern gekennzeichnet, welche sich auf die Numerierung der obenstehend aufgelisteten Verbindungen beziehen.

Beispiel 1 (Verbindung Nr. 2)

In einer Rührapparatur werden 500 g Eisessig, 182 g (1,0 Mol) 2,2,6,6-Tetramethylpiperidin-4-hydroxi-4-nitril und 200 g (1,4 Mol) Lävulinsäureethylester vorgelegt, worauf man bei ca. 20°C unter Kühlung 100 g (3 Mol) Chlorwasserstoff einleitet. Es wird bei 50°C unter Durchleiten eines schwachen HCl-Stromes ca. 16 Stunden gerührt. Anschliessend wird i. Vak. die Essigsäure abdestilliert, der Rückstand in 200 ml Aceton aufgenommen und das ausgefallene Hydrochlorid der gewünschten Verbindung abgenutscht. Nach Neutralisation des Salzes mit Ammoniakwasser oder Natriumcarbonatlösung fällt ein farbloser Feststoff an, welcher aus Heptan umkristallisiert wird.
Ausbeute: 160 g = 48% d.Th.; Fp. 150°C.

Beispiele 2 und 3

Man arbeitet wie in Beispiel 1 unter Einsatz desselben Nitrils und erhält durch Umsetzen mit Acetessigsäureethylester Verbindung Nr. 1 vom Fp. 141°C; (Beispiel 2), durch Umsetzen mit Cyclohexanon-2-propionsäurepropylester Verbindung Nr. 4 vom Fp. 185°C; (Beispiel 3).

Beispiel 4 (Verbindung Nr. 5)

In einer Rührapparatur werden in 80 ml abs. Mesitylen 13,0 g (0,04 Mol) der Verbindung nach Beispiel 1, 2,3 g (0,02 Mol) Hexamethylendiamin und ca. 0,2 g $LiNH_2$ bei 100°C gerührt, wobei Reaktionsethanol (1,8 g) abdestilliert. Die Reaktion ist nach 6 Stunden beendet. Es wird heiss filtriert, das Filtrat i. Vak. zur Trockene eingeengt und der Rückstand mit Ether verrührt. Der ausgefallene Feststoff wird abgenutscht.
Ausbeute: 9,0 g, Fp. 97 bis 99°C.

Beispiel 5 (Verbindung Nr. 9)

10 g der Verbindung nach Beispiel 3 werden bei 40°C in 100 ml konz. $NH_3$ 4 Stunden gerührt und abgenutscht.
Ausbeute: 9 g; Fp. 290°C.

Beispiel 6

Dieses Beispiel zeigt die Flüchtigkeit der neuen Stabilisatoren im Vergleich zu Produkten des nächstens Standes der Technik.

Die Flüchtigkeiten wurden in einer Apparatur zur thermogravimetrischen Analyse bestimmt. Gleiche Mengen (500 mg) der erfindungsgemässen Verbindungen und der Vergleichssubstanzen wurden dazu in Stickstoffatmosphäre mit einer Aufheizgeschwindigkeit von 2 K/min. bis auf 300°C erhitzt und der Substanzverlust in $mg/cm^2$ Probenoberfläche gemessen. Die Ergebnisse zeigt nachstehende Tabelle:

| Stabilisator nach Beispiel | Gewichtsverlust in $mg/cm^2$ beim Erreichen von ... °C | | | |
|---|---|---|---|---|
| | 220 | 260 | 300 | 10 min. bei 300 |
| 1 | 3,95 | 15,96 | 71,1 | 107,44 |
| 4 | 0,0 | 0,95 | 7,09 | 12,01 |

| Stabilisator nach Beispiel | Gewichtsverlust in mg/cm² beim Erreichen von ... °C | | | |
|---|---|---|---|---|
| | 220 | 260 | 300 | 10 min. bei 300 |
| 5 | 0,63 | 2,37 | 12,64 | 15,80 |
| Vergleich 1) | 6,64 | 85,82 | +) | – |
| Vergleich 2) | 0,79 | 3,63 | 13,27 | 20,22 |

1) Verbindung nach Beispiel 15 der DE-PS 2 606 026
2) Verbindung nach Beispiel 4 der DE-A 2 933 732
+) Bei 290°C gesamte Substanz verdampft

Beispiel 7

Eine in einem Laborschnellmischer hergestellte Mischung aus
100 Gew.-T. Polypropylen (®Hostalen PPK 1060 der Hoechst AG vom Schmelzindex MFI 190/5 = 2,0/10 min., bestimmt nach DIN 53 535),
0,2 Gew.-T. Calciumstearat,
0,1 Gew.-T. Pentaerythrityl-tetrakis-3-(3,5-di-tert.-butyl)-4-hydroxiphenyl)-propionat und
0,3 Gew.-T. des zu prüfenden Stabilisators
wird zu Granulat verarbeitet. Das derart stabilisierte Material schmilzt man sodann in einem Laborextruder unter den üblichen Verarbeitungsbedingungen auf und verspinnt es über eine Spinnpumpe mit Achtfachspinnkopf zu Monofilamenten. Diese werden anschliessend im Verhältnis 1:3 nachverstreckt und zu Garn von 40 dtex texturiert, welches man zu Prüfgeweben verarbeitet.

Die Gewebeproben werden auf einen gelochten Rahmen so aufgespannt, dass eine freie Öffnung von ca. 15,5 mm Durchmesser bestehen bleibt. In dieser Form werden die Prüflinge in einem Xenotest X-1200-Gerät der Firma Original Hanau Quarzlampen GmbH unter Bestrahlung mit Wechsellicht künstlich bewittert. Die Strahlungsintensität wurde durch UV-Filter (Spezialfilterglas d = 1,7 mm) moduliert. Die Lichtbeständigkeit wurde nach DIN 53 387 (17 min. Trockenperiode, 3 min. Beregnen, Schwarztafeltemperatur 45°C, relative Luftfeuchtigkeit während der Trockenperiode 70 bis 75%) geprüft. In bestimmten Zeitabständen werden die Gewebe zentrisch mit einem Gewicht von 6 mm Durchmesser und einem Druck von 0,1 N/m² belastet. Als Prüfendpunkt gilt das Durchbrechen des Gewichtes.

| Stabilisator nach Beispiel | Belichtungszeit in h |
|---|---|
| 1 | 600 |
| 4 | 800+) |
| 5 | 800+) |
| ohne Stabilisator | 280 |
| Vergleich1) | 500 |
| Vergleich2) | 800 |

+) noch nicht durchgebrochen
1), 2) Vergleichssubstanzen wie in Beispiel 6

**Patentansprüche für die Vertragsstaaten BE, CH, DE, FR, GB, IT, LI, NL, SE**

1. Polyalkyldiazaspirodecanderivate der Formel (I),

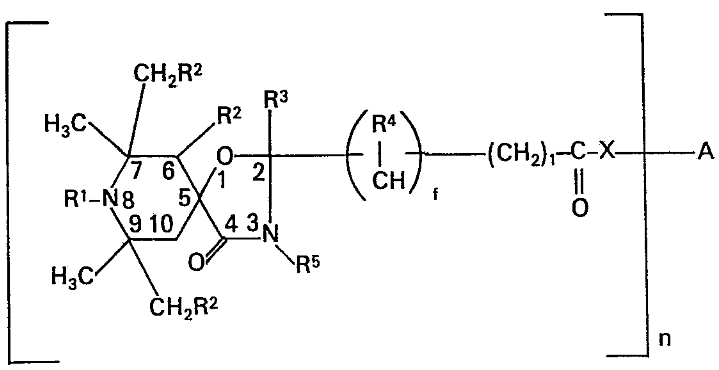

(I),

in der

$R^1$ Wasserstoff, Sauerstoff, $C_1$- bis $C_{12}$-Alkyl, das durch OH substituiert sein kann, oder 2,3-Dihydroxipropyl ist,

$R^2$ Wasserstoff oder $C_1$- bis $C_5$-Alkyl darstellt,

$R^3$ für $C_1$- bis $C_5$-Alkyl, das durch $-COOH$, $-COOCH_3$ oder $-COOC_2H_5$ substituiert sein kann, oder Phenyl steht und

$R^4$ dann Wasserstoff ist, oder

$R^3$ und $R^4$ zusammen mit den sie bindenden C-Atomen einen $C_5$- bis $C_{12}$-Cycloalkanring bilden,

f 0 oder 1 ist,

1 die Bedeutung einer ganzen Zahl von 0 bis 10 hat,

n eine ganze Zahl von 1 bis 10 bedeutet,

$R^5$ für Wasserstoff, für $C_1$- bis $C_{12}$-Alkyl, das in 2-Stellung durch OH substituiert sein kann, oder für eine Gruppe der Formel $-(CH_2)_kCOOR^6$, in welcher k 1 oder 2 und $R^6$ eine $C_1$- bis $C_{18}$-Alkylgruppe ist, steht,

X -0- oder

$$\begin{array}{c} R^7 \\ | \\ -N- \end{array}$$

wobei $R^7$ die unten für A bei m = 1 und n = 1 angegebenen Bedeutungen hat, darstellt,

A bei n = 1 für Wasserstoff, $C_1$- bis $C_{18}$-Alkyl, $C_3$- bis $C_7$-Alkenyl, $C_7$- bis $C_9$-Phenylalkyl, $C_5$- bis $C_{12}$-Cycloalkyl oder 2,2,6,6-Tetramethyl-4-piperidinyl, steht,

bei n = 2 ein $C_2$- bis $C_{30}$-Alkylenrest, ein $C_4$- bis $C_8$-Alkenylenrest oder ein Mono-, Di- oder Tricycloalkylenrest mit 6 bis 18 Kohlenstoffatomen ist,

bei n = 3 einen $C_3$- bis $C_8$-Alkantriylrest, einen Rest der Formel

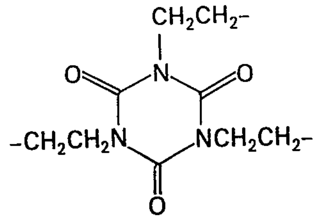

oder eine Gruppe, die zusammen mit X einen Rest der Formel

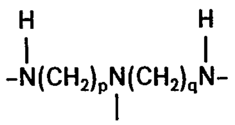

bildet, worin p und q gleich oder verschieden und 2 oder 3 sind, darstellt,

bei n = 4 eine $C_4$- bis $C_8$-Alkantetraylgruppe oder mit X zusammen einen Rest der Formel

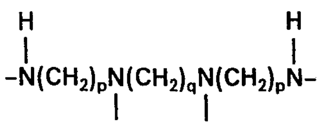

mit p = 2 oder 3 und q = 2 ist, und

bei n >4 einen $C_5$- bis $C_{10}$-Alkanrest, der eine Ethergruppe enthalten kann, oder mit X zusammen einen Polyethylenpolyaminrest bedeutet.

2. Verfahren zur Herstellung der Verbindungen nach Formel (I) in Anspruch 1, dadurch gekennzeichnet, dass man ein Polyalkylpiperidincyanhydrin der Formel (II)

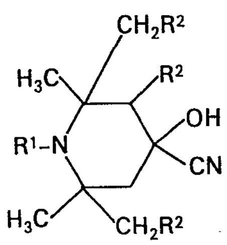 (II)

in welcher $R^1$ und $R^2$ die in Anspruch 1 angegebenen Bedeutungen haben, in einer $C_1$- bis $C_5$-Carbonsäure als Lösungsmittel in Gegenwart der, bezogen auf die Verbindung der Formel (II), 1,5- bis 5fach molaren Menge einer nicht oxidierenden anorganischen Säure bei 40 bis 120°C mit einer Verbindung der Formel (III)

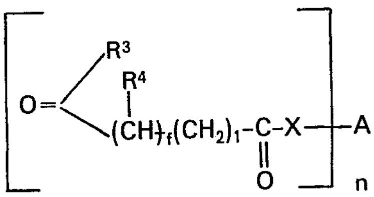 (III)

in der $R^3$, $R^4$, f, l, n und X sowie A die in Anspruch 1 angegebenen Bedeutungen haben, zur Umsetzung bringt, worauf man die dabei erhaltenen Salze in bekannter Weise in die freien Basen überführt und die Produkte, in denen n = 1, X = -O- und A = $-CH_3$ oder $-C_2H_5$ ist, gewünschtenfalls weiter umsetzt mit der äquivalenten Menge einer Verbindung der Formel (IV)

$$A(XH)_n \qquad (IV)$$

in der A, X und n die in Anspruch 1 angegebene Bedeutung haben, in einem inerten organischen Lösungsmittel und in Gegenwart eines basischen Katalysators, und - ebenfalls, sofern gewünscht - bei allen den Verbindungen der Formel (I), in denen $R^5$ = Wasserstoff ist, diesen durch Reagierenlassen mit der äquivalenten Menge eines Epoxids oder Alkylhalogenids substituiert.

3. Verwendung der Verbindungen nach Anspruch 1 zum Stabilisieren von synthetischen Polymeren.

4. Verwendung nach Anspruch 3, dadurch gekennzeichnet, dass das Polymere ein Polyolefin, Polyacrylat, Polymethacrylat oder ein Homo- oder Copolymerisat des Styrols ist.

5. Verfahren zum Stabilisieren von synthetischen Polymeren gegen den schädigenden Einfluss von Licht, dadurch gekennzeichnet, dass man den Polymeren, gegebenenfalls neben bisher bekannten, stabilisierend wirkenden Stoffen, 0,01 bis 5 Gewichtsteile, bezogen auf Polymeres, eines Stabilisators nach Anspruch 1 zusetzt.

6. Gegen UV-Zersetzung stabilisierte, synthetische Polymere, in denen 0,01 bis 5 Gewichtsteile, bezogen auf Polymeres, eines Stabilisators nach Anspruch 1 enthalten sind.

**Patentansprüche für den Vertragsstaat AT**

1. Verfahren zur Herstellung von Polyalkyldiazaspirodecanderivaten der Formel (I)

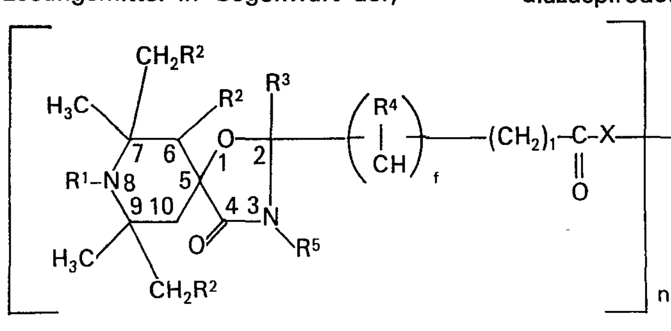 (I),

in der

$R^1$ Wasserstoff, Sauerstoff, $C_1$- bis $C_{12}$-Alkyl, das durch OH substituiert sein kann, oder 2,3-Dihydroxipropyl ist,

$R^2$ Wasserstoff oder $C_1$- bis $C_5$-Alkyl darstellt,

$R^3$ für $C_1$- bis $C_5$-Alkyl, das durch $-COOH$, $-COOCH_3$ oder $-COOC_2H_5$ substituiert sein kann, oder Phenyl steht und

$R^4$ dann Wasserstoff ist, oder

$R^3$ und $R^4$ zusammen mit den sie bindenden C-Atomen einen $C_5$- bis $C_{12}$-Cycloalkanring bilden,

f 0 oder 1 ist,

l die Bedeutung einer ganzen Zahl von 0 bis 10 hat,

n eine ganze Zahl von 1 bis 10 bedeutet,

$R^5$ für Wasserstoff, für $C_1$- bis $C_{12}$-Alkyl, das in 2-Stellung durch OH substituiert sein kann, oder für eine Gruppe der Formel $-(CH_2)_kCOOR^6$, in welcher k 1 oder 2 und $R^6$ eine $C_1$- bis $C_{18}$-Alkylgruppe ist, steht,

X -O- oder

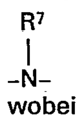

wobei $R^7$ die unten für A bei m = 1 und n = 1 angegebenen Bedeutungen hat, darstellt,

A bei n = 1 für Wasserstoff, $C_1$- bis $C_{18}$-Alkyl, $C_3$- bis $C_7$-Alkenyl, $C_7$- bis $C_9$-Phenylalkyl, $C_5$- bis $C_{12}$-Cycloalkyl oder 2,2,6,6-Tetramethyl-4-piperidinyl, steht,

bei n = 2 ein $C_2$- bis $C_{30}$-Alkylenrest, ein $C_4$- bis $C_8$-Alkenylenrest oder ein Mono-, Di- oder Tricycloalkylenrest mit 6 bis 18 Kohlenstoffatomen ist,

bei n = 3 einen $C_3$ bis $C_8$-Alkantriylrest, einen Rest der Formel

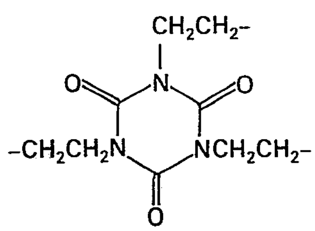

oder eine Gruppe, die zusammen mit X einen Rest der Formel

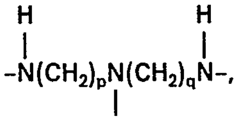

bildet, worin p und q gleich oder verschieden und 2 oder 3 sind, darstellt,

bei n = 4 eine $C_4$- bis $C_8$-Alkantetraylgruppe oder mit X zusammen einen Rest der Formel

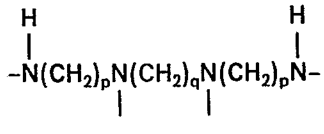

mit p = 2 oder 3 und q = 2 ist, und

bei n > 4 einen $C_5$- bis $C_{10}$-Alkanrest, der eine Ethergruppe enthalten kann, oder mit X zusammen einen Polyethylenpolyaminrest bedeutet, dadurch gekennzeichnet, dass man ein Polyalkylpiperidincyanhydrin der Formel (II)

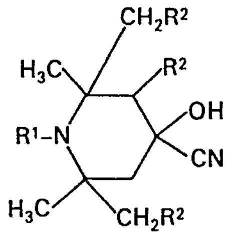

(II)

in welcher $R^1$ und $R^2$ die oben angegebenen Bedeutungen haben, in einer $C_1$- bis $C_5$-Carbonsäure als Lösungsmittel in Gegenwart der, bezogen auf die Verbindung der Formel (II), 1,5- bis 5fach molaren Menge einer nicht oxidierenden anorganischen Säure bei 40 bis 120°C mit einer Verbindung der Formel (III)

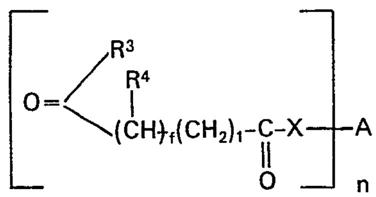

(III)

in der $R^3$, $R^4$, f, l, n und X sowie A die oben angegebenen Bedeutungen haben, zur Umsetzung bringt, worauf man die dabei erhaltenen Salze in bekannter Weise in die freien Basen überführt und die Produkte, in denen n = 1, X = -O- und A = $-CH_3$ oder $-C_2H_5$ ist, gewünschtenfalls weiter umsetzt mit der äquivalenten Menge einer Verbindung der Formel (IV)

$$A(XH)_n \qquad (IV),$$

in der A, X und n die oben angegebene Bedeutung haben, in einem inerten organischen Lösungsmittel und in Gegenwart eines basischen Katalysators, und - ebenfalls, sofern gewünscht - bei allen den Verbindungen der Formel (I), in denen $R^5$ = Wasserstoff ist, diesen durch Reagierenlassen mit der äquivalenten Menge eines Epoxids oder Alkylhalogenids substituiert.

2. Verwendung der nach Anspruch 1 erhaltenen Verbindungen zum Stabilisieren von synthetischen Polymeren.

3. Verwendung nach Anspruch 2, dadurch gekennzeichnet, dass das Polymere ein Polyolefin, Polyacrylat, Polymethacrylat oder ein Homo- oder Copolymerisat des Sryrols ist.

4. Verfahren zum Stabilisieren von synthetischen Polymeren gegen den schädigenden Einfluss von Licht, dadurch gekennzeichnet, dass man den Polymeren, gegebenenfalls neben bisher bekannten, stabilisierend wirkenden Stoffen, 0,01 bis 5 Gewichtsteile, bezogen auf Polymeres, eines nach Anspruch 1 erhaltenen Stabilisators zusetzt.

5. Gegen UV-Zersetzung stabilisierte, synthetische Polymere, in denen 0,01 bis 5 Gewichtsteile, bezogen auf Polymeres, eines nach Anspruch 1 hergestellten Stabilisators enthalten sind.

**Claims for the Contracting States: BE, CH, DE, FR, GB, IT, LI, NL, SE**

1. A polyalkyldiazaspirodecane derivative of the formula (I)

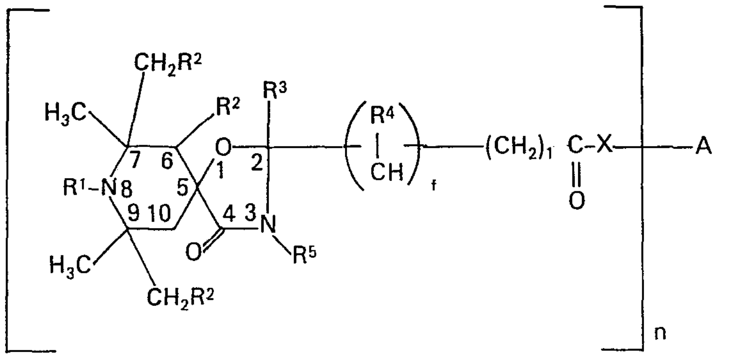

(I),

in which $R^1$ is hydrogen, oxygen, $C_1$ to $C_{12}$ alkyl which can be substituted by OH, or 2,3-dihydroxypropyl, $R^2$ represents hydrogen or $C_1$ to $C_5$ alkyl, $R^3$ represents $C_1$ to $C_5$ alkyl which can be substituted by $-COOH$, $-COOCH_3$ or $-COOC_2H_5$, or represents phenyl and $R^4$ is then hydrogen, or $R^3$ and $R^4$, together with the carbon atoms linking them, form a $C_5$ to $C_{12}$ cycloalkane ring, f is 0 or 1, 1 denotes an integer from 0 to 10, n denotes an integer from 1 to 10, $R^5$ represents hydrogen, or $C_1$ to $C_{12}$ alkyl which can be substituted by OH in the 2-position, or represents a group of the formula $-(CH_2)_kCOOR^6$ in which k is 1 or 2 and $R^6$ is a $C_1$ to $C_{18}$ alkyl group, X represents -O- or

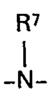

in which $R^7$ has the meanings indicated for A if n = 1, and, if n = 1, A represents hydrogen, $C_1$ to $C_{18}$ alkyl, $C_3$ to $C_7$ alkenyl, $C_7$ to $C_9$ phenylalkyl, $C_5$ to $C_{12}$ cycloalkyl or 2,2,6,6-tetramethyl-4-piperidinyl, if n = 2, A is a $C_2$ to $C_{30}$ alkylene radical, a $C_4$ to $C_8$ alkenylene radical or a monocycloalkylene, dicycloalkylene or tricycloalkylene radical having 6 to 18 carbon atoms, if n = 3, A represents a $C_3$ to $C_8$ alkanetriyl radical, a radical of the formula

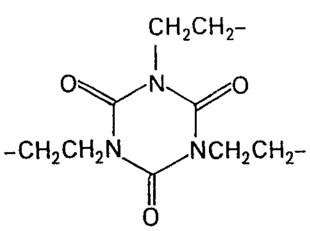

or a group which, together with X, forms a radical of the formula

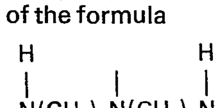

in which p and q are identical or different and are 2 or 3, if n = 4, A denotes a $C_4$ to $C_8$ alkanetetrayl group or, together with X, denotes a radical of the formula

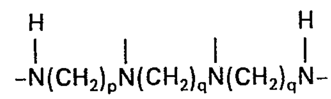

in which p is 2 or 3 and q is 2, and, if n > 4, A denotes a $C_5$ to $C_{10}$ alkane radical which can contain an ether group, or, together with X, denotes a polyethylenepolyamine radical.

2. A process for the preparation of a compound according to formula (I) in claim 1, which comprises reacting a polyalkylpiperidine cyanohydrin or the formula (II)

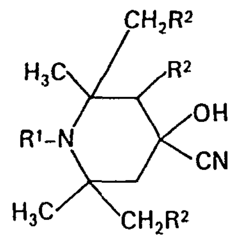

(II)

in which $R^1$ and $R^2$ have the meanings indicated in claim 1, in a $C_1$ to $C_5$ carboxylic acid as the solvent and in the presence of a 1.5-molar to 5-molar amount, relative to the compound of the formula (II), of a non-ozidizing inorganic acid at 40 to 120°C, with a compound of the formula (III)

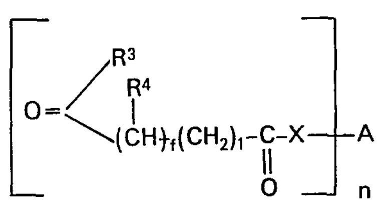

(III)

in which $R^3$, $R^4$, f, l, m, n and X and also A, have the meanings indicated in claim 1, then converting the salts obtained thereby into the free bases

in a known manner and, if desired, reacting products in which n is l, X is -O- and A is $-CH_3$ or $-C_2H_5$, further with an equivalent amount of a compound of the formula (IV)

$$A(XH)_n \qquad (IV)$$

in which A, X and n have the meaning indicated in claim 1, in an inert organic solvent and in the presence of a basic catalyst, and - also, if desired, - in the case of all the compounds of the formula (I) in which $R^5$ is hydrogen, substituting the latter by reacting them with an equivalent amount of an epoxide or alkyl halide.

3. The use of a compound as claimed in claim 1 for stabilizing synthetic polymers.

4. The use as claimed in claim 3, wherein the polymer is a polyolefin, polyacrylate, polymethacrylate or a homopolymer or copolymer of styrene.

5. A process for stabilizing synthetic polymers against the harmful effects of light, which comprises adding to the polymers, if appropriate together with hitherto known substances having a stabilizing action, 0.01 to 5 party by weight, relative to the polymer, of a stabilizer as claimed in claim 1.

6. Synthetic polymers which have been stabilized against decomposition by UV and which contain 0.01 to 5 parts by weight, relative to the polymer, of a stabilizer as claimed in claim 1.

**Claims for the Contracting State: Austria**

1. A process for the preparation of polyalkyldiazaspirodecane derivatives of the formula (I)

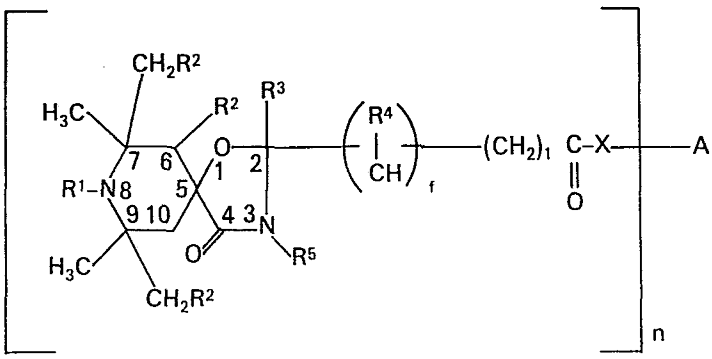 (I),

in which $R^1$ is hydrogen, oxygen, $C_1$ to $C_{12}$ alkyl which can be substituted by OH, or 2,3-dihydroxypropyl, $R^2$ represents hydrogen or $C_1$ to $C_5$ alkyl, $R^3$ represents $C_1$ to $C_5$ alkyl which can be substituted by $-COOH$, $-COOCH_3$ or $-COOC_2H_5$, or represents phenyl and $R^4$ is then hydrogen, or $R^3$ and $R^4$, together with the carbon atoms linking them, form a $C_5$ to $C_{12}$ cycloalkane ring, f is 0 or 1, 1 denotes an integer from 0 to 10, n denotes an integer from 1 to 10, $R^5$ represents hydrogen, or $C_1$ to $C_{12}$ alkyl which can be substituted by OH in the 2-position, or represents a group of the formula $-(CH_2)_kCOOR^6$ in which k is 1 or 2 and $R^6$ is a $C_1$ to $C_{18}$ alkyl group, X represents -O- or

$R^7$
|
-N-

in which $R^7$ has the meanings indicated for A if n = 1, and, if n = 1, A represents hydrogen, $C_1$ to $C_{18}$ alkyl, $C_3$ to $C_7$ alkenyl $C_7$ to $C_9$ phenylalkyl, $C_5$ to $C_{12}$ cycloalkyl or 2,2,6,6-tetramethyl-4-piperidinyl, if n = 2, A is a $C_2$ to $C_{30}$ alkylene radical, a $C_4$ to $C_8$ alkenylene radical or a monocycloalkylene, dicycloalkylene or tricycloalkylene radical having 6 to 18 carbon atoms, if n = 3, A represents a $C_3$ to $C_8$ alkanetriyl radical, a radical of the formula

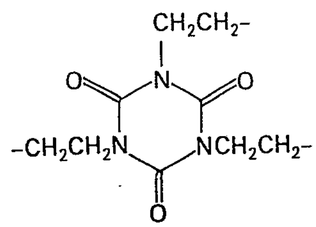

or a group which, together with X, forms a radical of the formula

H H
| | |
$-N(CH_2)_pN(CH_2)_qN-$

in which p and q are identical or different and are 2 or 3, if n = 4, A denotes a $C_4$ to $C_8$ alkanetetrayl group or, together with X, denotes a radical of the formula

H H
| | | |
$-N(CH_2)_pN(CH_2)_qN(CH_2)_qN-$

in which p is 2 or 3 and q is 2, and, if n >4, A denotes a $C_5$ to $C_{10}$ alkane radical which can contain an ether group, or, together with X, denotes a polyethylenepolyamine radical, which comprises reacting a polyalkylpiperidine cyanohydrin of the formula (II)

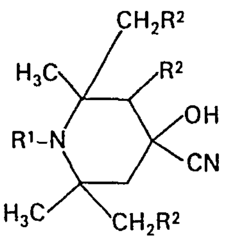

(II)

in which $R^1$ and $R^2$ have the meanings indicated in claim 1, in a $C_1$ to $C_5$ carboxylic acid as the solvent and in the presence of a 1.5-molar to 5-molar amount, relative to the compound of the formula (II), of a non-ozidizing inorganic acid at 40 to 120°C, with a compound of the formula (III)

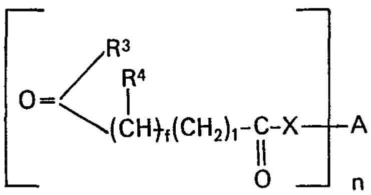

(III)

in which $R^3$, $R^4$, f, l, m, n and X and also A, have the meanings indicated above, then converting the salts obtained thereby into the free bases in a known manner and, if desired, reacting products in which n is l, X is -O- and A is $-CH_3$ or $-C_2H_5$, further with an equivalent amount of a compound of the formula (IV)

$A(XH)_n$ (IV)

in which A, X and n have the meaning indicated above in an inert organic solvent and in the presence of a basic catalyst, and - also, if desired, - in the case of all the compounds of the formula (I) in which $R^5$ is hydrogen, substituting the latter by reacting them with an equivalent amount of an epoxide or alkyl halide.

2. The use of a compound as claimed in claim 1 for stabilizing synthetic polymers.

3. The use as claimed in claim 2, wherein the polymer is a polyolefin, polyacrylate, polymethacrylate or a homopolymer or copolymer of styrene.

4. A process for stabilizing synthetic polymers against the harmful effects of light, which comprises adding to the polymers, if appropriate together with hitherto known substances having a stabilizing action, 0.1 to 5 parts by weight, relative to the polymer, of a stabilizer as claimed in claim 1.

5. Synthetic polymers which have been stabilized against decomposition by UV and which contain 0.01 to 5 parts by weight, relative to the polymer, of a stabilizer as claimed in claim 1.

**Revendications pour les Etats contractants: BE, CH, DE, FR, GB, IT, LI, NL, SE**

1. Polyalkyldiazaspirodécanes de formule (I) ci-dessous:

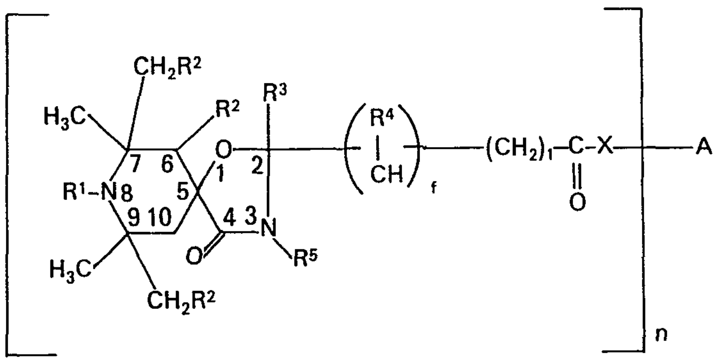

(I),

dans laquelle

$R^1$ représente l'hydrogène, l'oxygène, un alkyle en $C_1$ à $C_{12}$ pouvant être hydroxylé ou un groupe 2,3-dihydroxypropyle,

$R^2$ représente l'hydrogène ou un alkyle en $C_1$ à $C_5$.

$R^3$ un alkyle en $C_1$ à $C_5$ pouvant avoir comme substituant un groupe $-COOH$, $-COOCH_3$ ou $-COOC_2H_5$, ou un groupe phényle, et

$R^4$ représente l'hydrogène ou bien

$R^3$ et $R^4$ forment ensemble et avec les atomes de carbone qui les lient un cycle de cycloalcane en $C_5$ à $C_{12}$,

f est le nombre 0 ou 1,

l est un entier de 0 à 10,

n est un entier de l à 10,

$R^5$ représente l'hydrogène, un alkyle en $C_1$ à $C_{12}$ pouvant avoir un hydroxyle à la position 2, ou un groupe $-(CH_2)_kCOOR^6$, k étant le nombre 1 ou 2 et

$R^6$ un alkyle en $C_1$ à $C_{18}$,

X désigne -O- ou

$$\overset{R^7}{\underset{|}{}}\!\!-N-$$

$R^7$ ayant les significations qui sont données ci-après pour A dans le cas où n = 1, et

A représente:

si n = 1, l'hydrogène, un alkyle en $C_1$ à $C_{18}$, un alcényle en $C_3$ à $C_7$, un phénylalkyle en $C_7$ à $C_9$, un cycloalkyle en $C_5$ à $C_{12}$ ou un groupe 2,2,6,6-tétraméthyl-4-pipéridinyle,

si n = 2, un alkylène en $C_2$ à $C_{30}$, un alcénylène en $C_4$ à $C_8$ ou bien un mono-, di-, ou tricycloalkylène en $C_6$ à $C_{18}$,

si n = 3, un alcane-triyle en $C_3$ à $C_8$, un radical

de formule:

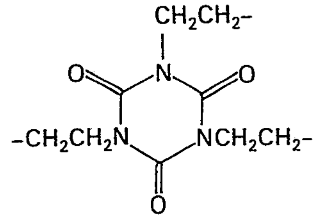

ou un groupe formant avec X un groupe de formule

$$-\overset{H}{\overset{|}{N}}(CH_2)_pN(CH_2)_q\overset{H}{\overset{|}{N}}-,$$

p et q étant chacun le nombre 2 ou 3 et pouvant être égaux ou non,

si n = 4, un groupe alcane-tétrayle en $C_4$ à $C_8$, ou bien forme avec X un groupe

$$-\overset{H}{\overset{|}{N}}(CH_2)_pN(CH_2)_qN(CH_2)_p\overset{H}{\overset{|}{N}}-$$

p étant le nombre 2 ou 3 et q le nombre 2, et

si n est supérieur à 4, un radical d'alcane en $C_5$ à $C_{10}$ pouvant comporter un groupe éther ou bien forme avec X un radical de polyéthylène-polyamine.

2. Procédé de préparation des composés de formule (I) selon la revendication 1, caractérisé en ce que l'on fait réagir une polyalkylpipéridyl-cyanhydrine de formule (II):

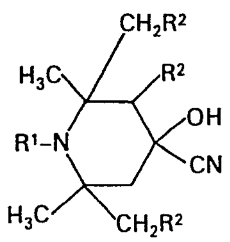

(II)

dans un acide carboxylique en $C_1$ à $C_5$ comme solvant et en présence, par rapport au composé de formule (II), d'une proportion 1,5 à 5 fois molaire d'un acide minéral non oxydant, à des températures de 40 à 120°C, avec un composé de formule (III):

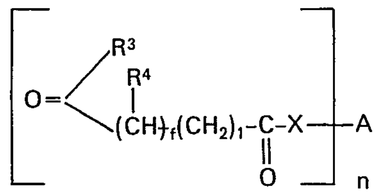

(III)

puis on transforme de manière connue les sels ainsi formés en bases libres, et le cas échéant on fait ensuite réagir les produits obtenus dans lesquels n = 1, X = -O- et A = $-CH_3$ ou $-C_2H_5$, avec la quantité équivalente d'un composé de formule (IV):

$$A\text{-}(XH)_n \qquad (IV)$$

dans un solvant organique inerte et en présence d'un catalyseur basique, et, de même si on le souhaite, pour tous les composés de formule (I) dans lesquels $R^5$ est l'hydrogène, on remplace cet hydrogène par un substituant par réaction avec la quantité équivalente d'un époxyde ou d'un halogénure d'alkyle.

3. L'utilisation des composés selon la revendication 1 pour stabiliser des matières polymères synthétiques.

4. Utilisation selon la revendication 3 caractérisée en ce que la matière polymère est une polyoléfine, un polyacrylate ou un polyméthacrylate ou bien un homopolymère ou un copolymère du styrène.

5. Procédé de stabilisation de polymères synthétiques contre l'action de la lumière, procédé caractérisé en ce que l'on ajoute à ces polymères, éventuellement en plus de matières stabilisantes déjà connues, de 0,01 à 5 parties en poids, par rapport au polymère, d'un stabilisant selon la revendication 1.

6. Polymères synthétiques stabilisés contre les dégradations par l'ultra-violet, qui contiennent de 0,01 à 5 parties en poids d'un stabilisant selon la revendication 1, par rapport au polymère.

**Revendications pour l'Etat contractant: AT**

1. Procédé de préparation de polyalkyldiazaspirodécanes de formule (I) ci-dessous:

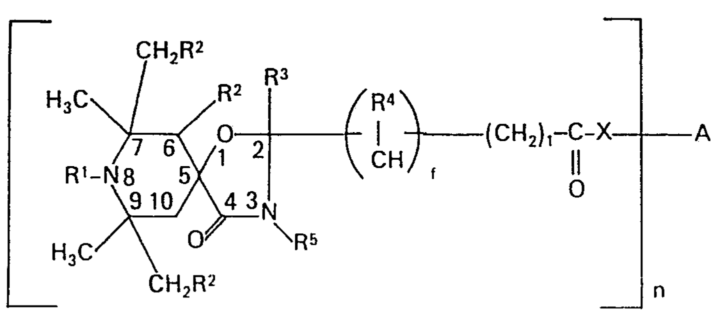

(I),

dans laquelle

$R^1$ représente l'hydrogène, l'oxygène, un alkyle en $C_1$ à $C_{12}$ pouvant être hydroxylé ou un groupe 2,3-dihydroxypropyle,

$R^2$ représente l'hydrogène ou un alkyle en $C_1$ à $C_5$, $R^3$ un alkyle en $C_1$ à $C_5$ pouvant avoir comme substituant un groupe $-COOH$, $-COOCH_3$ ou $-COOC_2H_5$, ou un groupe phényle, et
$R^4$ représente l'hydrogène ou bien
$R^3$ et $R^4$ forment ensemble et avec les atomes de carbone qui les lient un cycle de cycloalcane en $C_5$ à $C_{12}$,
f est le nombre 0 ou 1,
l est un entier de 0 à 10,
n est un entier de 1 à 10,
$R^5$ représente l'hydrogène, un alkyle en $C_1$ à $C_{12}$ pouvant avoir un hydroxyle à la position 2, ou un groupe $-(CH_2)_kCOOR^6$, k étant le nombre 1 ou 2 et $R^6$ un alkyle en $C_1$ à $C_{18}$,
X désigne -O- ou

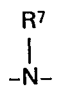

$R^7$ ayant les significations qui sont données ci-après pour A dans le cas où n = 1, et
A représente:
si n = 1, l'hydrogène, un alkyle en $C_1$ à $C_{18}$, un alcényle en $C_3$ à $C_7$, un phénylalkyle en $C_7$ à $C_9$, un cycloalkyle en $C_5$ à $C_{12}$ ou un groupe 2,2,6,6-tétraméthyl-4-pipéridinyle,
si n = 2, un alkylène en $C_2$ à $C_{30}$, un alcénylène en $C_4$ à $C_8$ ou bien un mono-, di-, ou tricycloalkylène en $C_6$ à $C_{18}$,
si n = 3, un alcane-triyle en $C_3$ à $C_8$, un radical de formule:

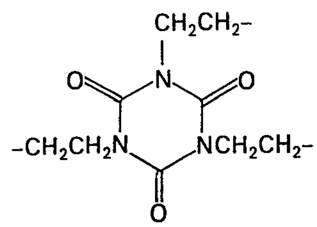

ou un groupe formant avec X un groupe de formule

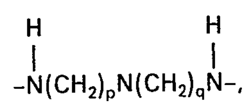

p et q étant chacun le nombre 2 ou 3 et pouvant être égaux ou non,
si n = 4, un groupe alcane-tétrayle en $C_4$ à $C_8$, ou bien forme avec X un groupe

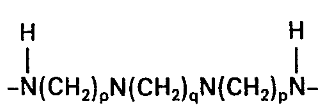

p étant le nombre 2 ou 3 et q le nombre 2, et
si n est supérieur à 4, un radical d'alcane en $C_5$ à $C_{10}$ pouvant comporter un groupe éther, ou bien forme avec X un radical de polyéthylène-polyamine, procédé caractérisé en ce que l'on fait réagir une polyalkylpipéridylcyanhydrine de formule (II):

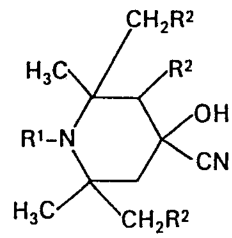

(II)

dans un acide carboxylique en $C_1$ à $C_5$ comme solvant et en présence, par rapport au composé de formule (II), d'une proportion 1,5 à 5 fois molaire d'un acide minéral non oxydant, à des températures de 40 à 120°C, avec un composé de formule (III):

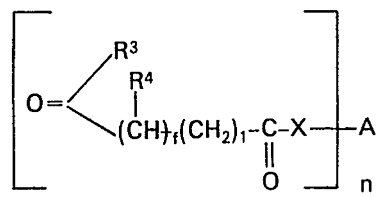

(III)

puis on transforme de manière connue les sels ainsi formés en bases libres, et le cas échéant on fait ensuite réagir les produits obtenus dans lesquels n = 1, X = -O- et A = $-CH_3$ ou $-C_2H_5$ avec la quantité équivalente d'un composé de formule (IV):

$$A{(XH)}_n \qquad (IV)$$

dans un solvant organique inerte et en présence d'un catalyseur basique, et de même si on le souhaite, pour tous les composés de formule (I) dans lesquels $R^5$ est l'hydrogène, on remplace cet hydrogène par un substituant par réaction avec la quantité équivalente d'un époxyde ou d'un halogénure d'alkyle.

2. L'utilisation des composés obtenus selon la revendication 1 pour stabiliser des matières polymères synthétiques.

3. Utilisation selon la revendication 2 caractérisée en ce que la matière polymère est une polyoléfine, un polyacrylate ou un polyméthacrylate ou bien un homopolymère ou un copolymère du styrène.

4. Procédé de stabilisation de polymères synthétiques contre l'action de la lumière, procédé caractérisé en ce que l'on ajoute à ces polymères, éventuellement en plus de matières stabilisantes déjà connues, de 0,01 à 5 parties en poids, par rapport au polymère, d'un stabilisant obtenu selon la revendication 1.

5. Polymères synthétiques stabilisés contre les dégradations par l'ultra-violet, qui contiennent de 0,01 à 5 parties en poids d'un stabilisant obtenu selon la revendication 1, par rapport au polymère.